(19) Europäisches Patentamt
European Patent Office
Office européen des brevets

(11) Numéro de publication : **0 461 012 B1**

(12) **FASCICULE DE BREVET EUROPEEN**

(45) Date de publication du fascicule du brevet : **20.04.94 Bulletin 94/16**

(51) Int. Cl.$^5$ : **C07D 207/09**, A61K 31/495, A61K 31/40

(21) Numéro de dépôt : **91401431.1**

(22) Date de dépôt : **03.06.91**

(54) **Dérivés de (1-phénylpyrrolidin-2-yl)méthylpipérazine, leur préparation et leur application en thérapeutique.**

(30) Priorité : **07.06.90 FR 9007067**

(43) Date de publication de la demande : **11.12.91 Bulletin 91/50**

(45) Mention de la délivrance du brevet : **20.04.94 Bulletin 94/16**

(84) Etats contractants désignés : **AT BE CH DE DK ES FR GB GR IT LI LU NL SE**

(56) Documents cités : **FR-A- 2 227 866**

(73) Titulaire : **SYNTHELABO**
**22, Avenue Galilée**
**F-92350 Le Plessis Robinson (FR)**

(72) Inventeur : **George, Pascal**
**19, rue des Quatre Vents**
**F-78730 St Arnoult en Yvelines (FR)**
Inventeur : **Menin, Jacques**
**Cité Balzac-D 114 / 118, rue Balzac**
**F-94400 Vitry/Seine (FR)**
Inventeur : **Merly, Jean-Pierre**
**11, Avenue Jules Guesde**
**F-92330 Sceaux (FR)**
Inventeur : **Bigg, Dennis**
**122, Avenue de Lavaur**
**F-81100 Castres (FR)**
Inventeur : **Obitz, Daniel**
**64, rue Velpeau**
**F-92160 Antony (FR)**
Inventeur : **Peynot, Michel**
**2, rue des Marguerites**
**F-94240 l'Hay-les-Roses (FR)**
Inventeur : **Veronique, Corinne**
**2, Place Paul Eluard / Appt. 194**
**F-94800 Villejuif (FR)**

(74) Mandataire : **Thouret-Lemaitre, Elisabeth et al**
**SYNTHELABO, Service Brevets, B.P. 72**
**F-92352 Le Plessis-Robinson Cédex (FR)**

**EP 0 461 012 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés de [(1-aryl-pyrrolidin-2-yl)méthyl]pipérazine, leur préparation et leur application en thérapeutique.

Les composés de l'invention répondent à la formule I donnée en annexe dans laquelle
Z représente un groupe de formule $N-R_1$ dans laquelle $R_1$ est soit un atome d'hydrogène, soit un groupe $(C_1-_3)$alkyle, soit un groupe de formule $Ar-C_nH_{2n}$ dans laquelle n représente 0,1 ou 2 et Ar représente un groupe phényle éventuellement substitué par un ou plusieurs atomes d'halogène et/ou radicaux $(C_{1-3})$alkyles ou $(C_{1-3})$alcoxy, soit un groupe de formule $COR_2$ dans laquelle $R_2$ représente le groupe $CH_3$, $C_6H_5$, $CH_2C_6H_5$ ou $OC_2H_5$.

Les composés de l'invention existent sous la forme de racémates ou d'énantiomères qui font partie de l'invention.

Les sels que forment les composés (I) avec les acides pharmaceutiquement acceptables font partie de l'invention.

Les composés préférés de l'invention sont les composés (I) dans lesquels Z représente un radical $N-(CH_2)_2-Ar$ où Ar représente un groupe phényle éventuellement substitué par un ou plusieurs atomes de fluor ou de chlore et/ou par un ou plusieurs radicaux méthyle ou méthoxy.

Les composés de l'invention peuvent être préparés selon le schéma réactionnel donné en annexe.
Selon l'invention

. soit on fait réagir, dans un solvant inerte tel que le toluène, à une température allant de 80 à 110°C, un ester dérivé de la proline de formule (II) avec le complexe triméthylaluminium-amine de formule (III), préparé au préalable dans un solvant inerte tel que le toluène, à une température allant de -20 à 20°C pour obtenir le composé (IV)

. soit on fait réagir la proline (V) avec un halogénure $C_6H_5Hal$ en présence soit de sel de Cu++ sous forme de CuO, soit de cuivre métallique, en milieu basique, par exemple $K_2CO_3$, dans un solvant aprotique, tel que le DMF ou la N-méthylpyrrolidone, à une température allant de 100 à 150°C, selon les conditions décrites dans la réaction de Ullmann (Synthesis 9(1974)) pour obtenir un acide de formule (VI) ; puis on effectue la transformation de l'acide (VI) en amide (IV), de manière classique, en traitant l'acide (VI) au moyen de carbonyldiimidazole dans un solvant, tel que le tétrahydrofurane (THF) ou le chlorure de méthylène ($CH_2Cl_2$), à une température allant de 20 à 40°C pour former l'imidazolide intermédiaire que l'on traite in situ par une amine de formule (III) dans laquelle Z a la signification donnée ci-dessus.

Enfin on réduit l'amide obtenu de formule (IV) au moyen d'hydrure de lithium et d'aluminium dans un solvant éthéré tel que l'éther ou le tétrahydrofurane (THF) à une température allant de 20 à 50°C en composé de formule (I).

Les esters de formule (II) peuvent être préparés selon la méthode décrite dans Khim. Farm. Zh 1970,4 (9), pages 27-31 (CA 74-012920).

Les composés de formule (I) dans laquelle Z = NH peuvent également être obtenus

1) soit par débenzylation des composés de formule (IV) dans laquelle $Z = N-CH_2-C_6H_5$ au moyen d'hydrogène en présence d'un catalyseur tel que le palladium absorbé sur un support inerte, à une température allant de 20 à 60°C, dans un solvant protique tel que l'éthanol ou l'acide acétique, sous une pression de 100 à 400 kPa suivie d'une réduction du composé de formule (IV) dans laquelle Z = NH ainsi obtenu, au moyen de $LiAlH_4$, dans un solvant éthéré tel que l'éther ou le THF, à une température allant de 20 à 50°C ;

2) soit par réaction des composés de formule (IV) dans laquelle $Z = N-CH_2-C_6H_5$ avec un chloroformiate d'alkyle, plus particulièrement le chloroformiate d'éthyle, dans un solvant chloré tel que $CH_2Cl_2$, à une température allant de 0 à 20°C, suivie d'une saponification des composés de formule (IV) dans laquelle $Z = NCO_2Et$, au moyen d'hydroxyde sodium dans de l'éthanol, à une température allant de 20 à 80°C et d'une réduction des composés de formule (IV) dans laquelle Z = NH par $LiAlH_4$ dans les mêmes conditions que ci-dessus.

Les composés de formule (I) dans laquelle Z représente un groupe de formule $NCOR_2$ peuvent également être obtenus par réaction des composés de formule (I) dans laquelle Z est NH avec un réactif de formule $R_2CO-Hal$ dans laquelle $R_2$ est défini comme ci-dessus et Hal représente un atome d'halogène, dans un solvant inerte tel que $CH_2Cl_2$, le toluène ou le DMF, éventuellement en présence d'une base minérale telle que $K_2CO_3$ ou organique telle que la triéthylamine (TEA), à une température allant de 20 à 100°C.

Les racémates peuvent être obtenus au départ de (D,L)-proline. Les énantiomères purs peuvent être obtenus soit en partant de (L)(-)-proline ou de (D)(+)-proline, soit par résolution du mélange racémique au moyen d'un agent de résolution tel qu'un acide optiquement actif.

Les exemples suivants illustrent la préparation des composés selon l'invention.

Les analyses et les spectres IR et RMN confirment la structure des composés.

Exemple 1.

[1-(benzyl)-4-(1-phénylpyrrolidin-2-yl)méthyl]pipérazine et son dichlorhydrate.

1.1. [1-(benzyl)-4-(1-phénylpyrrolidin-2-yl) carbonyl]pipérazine.

A une solution de 3,8 g (0,0526 mole) de triméthylaluminium (22 $cm^3$ d'une solution à 25 % dans de l'hexane) dans 40 $cm^3$ de toluène, sous argon, à 0-5°C, on ajoute, goutte à goutte, une solution de 9,55 g (0,054 mole) de 1-benzylpipérazine dans 20 $cm^3$ de toluène. Puis on porte le mélange à 50°C, ajoute lentement 7,43 g (0,0339 mole) de 1-phénylpyrrolidine-2-carboxylate d'éthyle dans 40 $cm^3$ de toluène. En fin d'addition on chauffe à 80-90°C, distille l'hexane et porte à reflux 3 heures. On obtient une solution limpide, orange que l'on refroidit et hydrolyse par 50 $cm^3$ d'eau. On filtre et rince l'insoluble à l'acétate d'éthyle, lave à l'eau après avoir décanté la phase organique et la sèche. On concentre la solution sous pression réduite, on obtient une huile jaune que l'on reprend dans un mélange cyclohexane/toluène (1/1). On filtre, triture la solution et obtient un solide blanc. m = 10,3 g. F = 99-100,5°C.

1.2. [1-(benzyl)-4-(1-phénylpyrrolidin-2-yl)méthyl]pipérazine et son dichlorhydrate.

Dans un ballon tricol de 1 l, on introduit sous argon, 1,65 g (0,0434 mole) de $LiAlH_4$ dans 75 $cm^3$ de THF sec, puis on ajoute à 0-5°C, 10,1 g (0,0289 mole) du composé obtenu sous 1.1. dans 200 $cm^3$ de THF sec. On laisse en contact 30 mn, puis porte à reflux pendant 6 heures. On traite le mélange par 15 $cm^3$ de NaOH à 6,5 %, en refroidissant.
On filtre la solution obtenue, rince l'insoluble au THF. On rassemble les phases organiques, sèche et concentre sous pression réduite. On obtient une huile jaune.
On prépare le chlorhydrate du composé en faisant réagir 9,7 g (0,0289 mole) de base et 580 $cm^3$ de HCl dans de l'isopropanol (0,1 m/l). On concentre la solution totalement, ajoute de l'AcOEt et triture. On fait recristalliser le solide blanc dans un mélange isopropanol/MeOH (1/1).
m = 10,8 g. F = 249-251°C. Rendement = 91,5 %.

Exemple 2.

1-[(1-phénylpyrrolidin-2-yl)méthyl]pipérazine et son hémifumarate.

2.1. 1-[(1-phénylpyrrolidin-2-yl)carbonyl]pipérazine.

Voie de synthèse 1.

On fait réagir 3,49 g (0,01 mole) du composé de l'exemple 1.1 dissous dans 100 ml d'éthanol, en présence de 0,5 g de Palladium/C 10 % et de 10 ml d'acide chlorhydrique 1N, avec de l'hydrogène gazeux sous une pression de 4 atm pendant 5 h à une température de 20 à 25°C. En fin de réaction, le catalyseur est filtré et le filtrat est concentré sous pression réduite. Le résidu est traité avec un excès d'ammoniaque et extrait au $CH_2Cl_2$. La phase organique est séchée sur $Na_2SO_4$, et concentrée. On obtient 2,5 g soit 96 % de solide blanc. F = 128-130°C.

Voie de synthèse 2.

4-[(1-phénylpyrrolidin-2-yl)carbonyl]pipérazine-1-carboxylate d'éthyle.
On introduit 53,0 g (0,152 mole) du composé de l'exemple 1.1. dans 500 $cm^3$ de $CH_2Cl_2$, sous argon, et on ajoute, à la température ambiante, 18,2 g (0,168 mole) de chloroformiate d'éthyle. On laisse agiter le mélange à la température ambiante pendant 72 heures.
On concentre la solution, reprend le résidu au $CH_2Cl_2$, lave à l'eau sodée, puis à l'eau, sèche et concentre. On fait recristalliser le solide obtenu dans un mélange cyclohexane/toluène (4/1). m = 42,7 g. Rendement = 84,9 %. F = 139,5-141,5°C.

1-[(1-phénylpyrrolidin-2-yl)carbonyl]pipérazine.

Dans un ballon tricol de 1 l, on introduit sous argon, 46,1 g (0,139 mole) du composé obtenu précédemment et 300 $cm^3$ d'éthanol. On porte le mélange à ébullition et ajoute 85 $cm^3$ (0,834 mole) d'une solution d'hydroxyde

de sodium.
On porte le mélange à ébullition pendant 18 heures.
On ajoute 700 $cm^3$ d'eau et extrait au $CH_2Cl_2$. On sèche la phase organique sur $MgSO_4$. On concentre sous pression réduite et obtient un solide blanc que l'on fait recristalliser dans du toluène.
m = 31,6 g. Rendement = 87,5 %. F = 128-130°C.

2.2. 1-[(1-phénylpyrrolidin-2-yl)méthyl]pipérazine et son hémifumarate.

Dans un ballon tricol de 1 l, on introduit sous argon, 5,84 g (0,154 mole) de $LiAlH_4$ dans 600 $cm^3$ de THF sec, puis ajoute à la température de 0-5°C, 26,6 g (0,103 mole) d'amide 2.1. On laisse en contact 30 mn puis porte le mélange à reflux pendant 2 heures. On traite ensuite par 70 $cm^3$ de NaOH à 6,5 %, en refroidissant. On filtre la suspension obtenue et lave l'insoluble à l'acétate d'éthyle. On sèche le filtrat sur $MgSO_4$ et concentre sous pression réduite. On obtient une huile jaune, m = 23,5 g, rendement : 93,3 %.
On prépare l'hémifumarate de ce composé en faisant réagir 5,0 g (0,0204 mole) de base dans 50 $cm^3$ d'éthanol et 1,19 g (0,0102 mole) d'acide fumarique dans 100 $cm^3$ d'éthanol. On concentre la solution sous pression réduite et fait recristalliser le résidu solide dans du méthanol.
m = 1,3 g. Rendement 22 %. F = 179,5-181°C.

Exemple 3

1-(2-phényléthyl)-4-[(1-phénylpyrrolidin-2-yl)méthyl]pipérazine et son dichlorhydrate.

3.1. 1-(2-phényléthyl)-4-[(1-phénylpyrrolidin-2-yl)carbonyl]-pipérazine.

A une solution de 3,8 g (0,0526 mole) de triméthylaluminium (22 $cm^3$ d'une solution à 25 % dans l'hexane) dans 40 $cm^3$ de toluène, sous argon, à 0-5°C, on ajoute, goutte à goutte, une solution de 10,3 g (0,0542 mole) de 1-(2-phényléthyl)pipérazine dans 20 $cm^3$ de toluène.
On porte le mélange à 50°C, ajoute goutte à goutte 7,43 g (0,0339 mole) de 1-phénylpyrrolidine-2-carboxylate d'éthyle dans 40 $cm^3$ de toluène. En fin d'addition, on chauffe le mélange à 80°C, distille l'hexane et porte à reflux 3 heures.
On refroidit la solution et hydrolyse par 50 $cm^3$ d'eau. On filtre la solution, rince l'insoluble à l'acétate d'éthyle, décante, lave à l'eau, sèche et concentre. On reprend l'huile dans du cyclohexane à chaud. On filtre et triture le résidu. On obtient un solide blanc.
m = 11,4 g. Rendement = 92,7 %. F = 123-124,5°C.

3.2. 1-(2-phényléthyl)-4-[(1-phénylpyrrolidin-2-yl)méthyl]pipérazine et son chlorhydrate.

Dans un ballon tricol de 500 $cm^3$, on introduit, sous argon, 1,25 g (0,0330 mole) de $LiAlH_4$ dans 50 $cm^3$ de THF sec puis on ajoute, à 0-5°C, 8,0 g (0,0220 mole) du composé 3.1. dans 100 $cm^3$ de THF sec. On laisse en contact 30 mn, puis on porte le mélange à reflux 7,5 heures. On traite la solution par 10 $cm^3$ de NaOH à 6,5 %, en refroidissant.
On filtre la suspension, rince l'insoluble au THF, sèche et concentre. On obtient une huile jaune.
On prépare le dichlorhydrate en faisant réagir 6,6 g (0,0189 mole) de base 3.2 et 380 $cm^3$ de HCl dans de l'isopropanol (0,1 m/l). On concentre la solution aux 3/4. On obtient un solide blanc que l'on filtre et fait recristalliser dans un mélange isopropanol/MeOH (1/3).
m = 6,9 g. Rendement = 74,2 %. F = 272-275°C.

Exemple 4.

1-(Phénylcarbonyl)-4-[(1-phénylpyrrolidin-2-yl)méthyl]pipérazine et son chlorhydrate.
Dans un ballon tricol de 500 $cm^3$, sous argon, on introduit 5,0 g (0,0204 mole) de 1-[(1-phénylpyrrolidin-2-yl)méthyl]pipérazine dans 75 $cm^3$ de $CH_2Cl_2$. On ajoute 7,2 g (0,0714 mole) de triéthylamine. On ajoute à la solution 3,0 g (0,0214 mole) de chlorure de benzoyle dans 40 $cm^3$ de $CH_2Cl_2$. On agite le mélange à la température ambiante pendant 1,5 heure. On traite la solution par 200 $cm^3$ d'eau, décante la phase organique, la lave à l'eau, la sèche et la concentre sous pression réduite. On obtient une huile que l'on chromatographie sur colonne de silice (éluant $CH_2Cl_2$/méthanol 99/1).
On prépare le chlorhydrate en faisant réagir 3,1 g (0,00887 mole) de base et 89 $cm^3$ de HCl dans de l'isopropanol (0,1 m/l).

On évapore la solution totalement. On obtient un solide que l'on fait recristalliser 2 fois dans un mélange isopropanol/-MeOH (4/1). F = 227-230°C. Rendement 32,9 %.

Exemple 5.

(S)(-)-1-(2-phényléthyl)-4-[(1-phénylpyrrolidin-2-yl)méthyl]-pipérazine et son dichlorhydrate.

5.1. Acide (S)(-)-1-phénylpyrrolidine-2-carboxylique.

Dans un ballon tricol de 2 l, on introduit 158 g (1,37 mole) de (L)(-)proline, 220 g (1,40 mole) de bromobenzène, 194 g (1,40 mole) de $K_2CO_3$ et 10 g de CuO dans 450 ml de DMF. On porte le mélange à la température de reflux pendant 37 heures. On laisse refroidir, ajoute 1 l d'eau, filtre sur celite, extrait les impuretés au $CH_2Cl_2$, filtre à nouveau et acidifie par HCl dilué jusqu'à pH 4,0.
On l'extrait au $CH_2Cl_2$, lave à l'eau, sèche et évapore le solvant sous pression réduite. On obtient un solide blanc. F = 80-81°C.

5.2. (S)(-)-1-(2-phényléthyl)-4[(1-phénylpyrrolidin-2-yl)carbonyl]-pipérazine.

A une solution de 14,2 g d'acide préparé en 5.1. dans 100 ml de THF, on ajoute 16,2 g (0,1 mole) de carbonyldiimidazole et laisse agiter 2 heures à 20°C puis 12 h à 40°C. On ajoute ensuite 14 g (0,074 mole) de 1-(2-phényl-éthyl)pipérazine et laisse agiter toute une nuit à 20°C puis 3 h à 50°C. On évapore le mélange à sec, reprend le résidu à l'eau contenant du bicarbonate de sodium, extrait au $CH_2Cl_2$, sèche la phase organique et concentre sous pression réduite.
On obtient une huile brune que l'on purifie par chromatographie sur colonne de silice (éluant : $CH_2Cl_2$-acétate d'éthyle 95/5, puis $CH_2Cl_2$-méthanol 95/5).
On récupère 10 g de produit brut que l'on dissout dans l'éther et reprécipite au pentane. On obtient 8 g de solide blanc (soit 30 % de rendement) que l'on utilise tel quel pour l'étape suivante.

5.3. (S)(-)-1-(2-phényléthyl)-4-[(1-phénylpyrrolidin-2-yl)méthyl]pipérazine et son dichlorhydrate.

Une solution de 6,9 g (0,019 mole) d'amide préparé selon 5.2. dans 100 ml de THF sec, est ajoutée à une suspension de 1,07 g (0,028 mole) de $LiALH_4$ dans 100 ml de THF sec. On porte le mélange au reflux pendant 3 h puis refroidit et hydrolyse par NaOH 1N. Il se forme un solide, la phase organique est décantée, diluée au $CH_2Cl_2$ et séchée. Les solvants sonst évaporés sous pression réduite. On obtient 7 g d'huile que l'on purifie par chromatographie sur colonne de silice (éluant : $CH_2Cl_2$ - acétate d'éthyle 97/3 puis $CH_2Cl_2$ - méthanol 95/5). On isole 6 g d'huile.
On prépare le dichlorhydrate au moyen de 5,43 g (0,0155 mole) de base et 311 ml d'acide chlorhydrique 0,1 N dans le 2-propanol, on fait recristalliser le sel dans du méthanol. On obtient 5,2 g de dichlorhydrate. F = 266-267°C,
$[\alpha]_D$= -15°(c = 0,2, éthanol).

Dans le tableau suivant sont représentés quelques composés de l'invention préparés à titres d'exemples.

Tableau

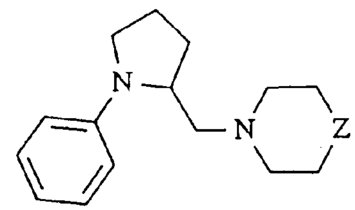

(I)

| N° | Z | Sel | F(°C) | $[\alpha]_D$ (°) |
|---|---|---|---|---|
| 1 | N-H | 1/2 fum. | 179,5-181 | d,l |
| 2 | $N-CH_3$ | HCl | 190,5-192 | d,l |
| 3 | $N-C_6H_5$ | HCl | 248-252 | d,l |
| 4 | $N-CH_2-C_6H_5$ | 2 HCl | 249-251 | d,l |
| 5 | $N-CH_2-(4-Cl-C_6H_4)$ | 2 HCl | 249-251,5 | d,l |
| 6 | $N-(CH_2)_2-C_6H_5$ | 2 HCl | 272-275 | d,l |
| 7 | $N-CH(CH_3)-C_6H_5$ | 2 fum. | 190-193 | d,l |
| 8 | $N-COCH_3$ | HCl | 250,5-253,5 | d,l |
| 9 | $N-CO_2C_2H_5$ | HCl | 226-228 | d,l |
| 10 | $N-COC_6H_5$ | HCl | 227-230 | d,l |

| N° | Z | Sel | F(°C) | $[\alpha]_D$ (°) |
|---|---|---|---|---|
| 11 | $N-(CH_2)_2-C_6H_5$ | 2 HCl | 266-267 | -15 (c=0,2,EtOH) |
| 12 | $N-(CH_2)_2-(4-CH_3O-C_6H_4)$ | 2 HCl | 258-260 | d,l |
| 13 | $N-(CH_2)_2-(4-Cl-C_6H_4)$ | 2 HCl | 247-249 | d,l |
| 14 | $N-(CH_2)_2-(3-CH_3O-C_6H_4)$ | 2 HCl | 235-237 | d,l |
| 15 | $N-(CH_2)_2-(4-F-C_6H_4)$ | 2 HCl | 250-253 | d,l |
| 16 | $N-(CH_2)_2-(2-CH_3O-C_6H_4)$ | 2 HCl | 235-237 | -16,7 (c=1,DMF) |
| 17 | $N-(CH_2)_2-(4-CH_3-C_6H_4)$ | 2 HCl | 255-257 | -34,5 (c=0,2,EtOH 95%) |
| 18 | $N-(CH_2)_2-(3-CH_3-C_6H_4)$ | 2 HCl | 250 (décomp.) | -19,2 (c=1,EtOH 95 %) |
| 19 | $N-(CH_2)_2-(2-CH_3-C_6H_4)$ | 2 HCl | 250 (décomp.) | -18,4 (c=1,EtOH 95 %) |
| 20 | $N-(CH_2)_2-(2-Cl-C_6H_4)$ | 2 HCl | 241-243 | -12,5 (c=1,EtOH 95 %) |

| N° | Z | Sel | F(°C) | $[\alpha]_D$ (°) |
|---|---|---|---|---|
| 21 | $N-(CH_2)_2-(3-Cl-C_6H_4)$ | 2 HCl | 245-248 | -21 (c=0,5,EtOH 95 %) |
| 22 | $N-(CH_2)_2-(3,4-diCH_3O-C_6H_3)$ | 2 fum. | 204-206 | -28 (c=0,1,EtOH 95 %) |
| 23 | $N-(CH_2)_2-(3,4-diCl-C_6H_3)$ | 2 mal. | 208-210 | -25 (c=0,1,EtOH 95 %) |
| 24 | $N-CO-CH_2-C_6H_5$ | HCl | 220-222 | d,l |
| 25 | $N-(CH_2)_2-(2-F-C_6H_4)$ | 2 HCl | 247-250 | -29 (c=0,1 EtOH 95 %) |
| 26 | $N-(CH_2)_2-(3-F-C_6H_4)$ | 2 HCl | 246-249 | -33 (c=0,1 EtOH 95 %) |
| 27 | $N-(CH_2)_2-C_6H_5$ | 2 HCl | 256-258 | +15,2 (c=0,2, EtOH) |

1/2 fum. = hémifumarate
2 fum. = difumarate
2 HCl = dichlorhydrate
2 mal. = maléate

Les composés de l'invention ont fait l'objet d'une série d'essais pharmacologiques qui ont mis en évidence leur intérêt comme substances à activités thérapeutiques.

Activité antiischémique

1. Les propriétés anti-ischémiques cérébrales des composés de l'invention ont été évaluées sur le test de l'ischémie cérébrale globale chez la souris.
L'ischémie cérébrale globale est induite chez la souris après un arrêt cardiaque provoqué par injection d'une solution saturée de $MgCl_2$ dans la veine caudale. Des études préliminaires ont montré que la chute de pression artérielle est similaire chez tous les animaux et que celle-ci est pratiquement nulle après 6.3 ± 0.8 s. L'arrêt cardiaque est suffisamment brutal pour que le chlorure de magnésium n'atteigne pas le lit capillaire cérébrovasculaire.
Le "temps de survie" est le temps qui sépare l'injection du chlorure de magnésium du dernier mouvement inspiratoire. La disparition complète des réserves énergétiques cérébrales ou, plus précisément, la perte

de l'autorythmicité des centres respiratoires cérébraux se traduit par un arrêt des mouvements thoraciques. Cet arrêt respiratoire survient environ 19 s après l'injection de chlorure de magnésium.
On mesure le temps de survie 10 minutes après une injection intrapéritonéale ou 30 minutes après l'administration par voie orale du composé à tester. La différence entre le temps de survie des animaux traités et le temps de survie des animaux témoins est reportée sur un graphique en fonction du logarithme de la dose (exprimée en base). La dose $ED_{3''}$ est la dose qui augmente le temps de survie des animaux traités de 3 secondes. Cette valeur est reproductible et toujours statistiquement significative.

Les doses $ED_{3''}$ des composés de l'invention se situent entre 10 et 200 mg/kg par voie intrapéritonéale et/ou par voie orale.
2. L'activité neuroprotectrice des composés de l'invention a été montrée dans un modèle d'ischémie focale par ligature de l'artère cérébrale moyenne chez la souris, selon une méthode analogue à celle décrite dans *Brain Research*, **522**, (1990), 290-307. Six jours après occlusion de l'artère cérébrale moyenne par électrocoagulation sous anesthésie à l'halothane, les souris sont à nouveau anesthésiées et le cortex cérébral ipsilatéral à l'occlusion est prélevé. Après homogénéisation du tissu, l'étendue de l'infarctus cérébral est évaluée par mesure de l'augmentation de la densité des sites benzodiazépiniques périphériques ($\omega_3$) à l'aide du composé [$^3$H]-PK 11195 de New England Nuclear. Les traitements sont administrés curativement aux temps 5 mn, 3 h, 6 h, 18 h et 24 h, par voie intrapéritonéale.

Avec les composés de l'invention, la réduction de la taille de la lésion va de 40 % jusqu'à 70 % (pour une dose allant de 0,1 à 30 mg/kg).

Activité anticonvulsivante

Les composés de l'invention inhibent les convulsions toniques induites chez la souris par un électrochoc supramaximal. Les composés étant administrés par voie intrapéritonéale 30 minutes, ou par voie orale 60 minutes, avant l'application sur la cornée d'un choc électrique (10 mA, 50Hz pendant 0,5s), on détermine graphiquement les $DA_{50}$, doses qui inhibent les effets convulsivants de l'électrochoc chez 50 % des animaux.

Pour les composés de l'invention les $DA_{50}$ se situent entre 10 et 100 mg/kg par voie intrapéritonéale.

Liaison spécifique aux sites sigma.

Les composés ont été soumis à un essai d'inhibition de la liaison de la [$^3$H]-(+)-3-(3-hydroxyphényl)-N-(1-propyl)pipéridine, ou [$^3$H]-(+)-3-PPP, aux récepteurs $\sigma$ du cerveau du rat, d'après le protocole décrit par Largent et coll., dans J. Pharmacol. Exp. Ther., 238, 739-748 (1986). On sacrifie des rats mâles Sprague-Dawley de 150 à 200 g, et on en homogénéise le cortex cérébral dans 25 volumes de tampon Tris-HCl à 50 mM (ph = 7,4 à 25°C) glacé, au moyen d'un appareil Ultra-Turrax™. On lave le mélange deux-fois en le centrifugeant pendant 10 mn à 45000 g et en remettant le culot en suspension dans du tampon frais. On dilue le culot lavé dans 20 volumes de tampon Tris(HCl à 50 mM pH = 8,0 à 22°C), et on fait incuber des fractions aliquotes de 75 µl dans un volume final de 250 µl à 2 nM de [$^3$H]-(+)-3PPP (activité spécifique : 90 Ci/mmole, d'origine New England nuclear), pendant 90 mn à 25°C, en l'absence ou en présence de substances compétitrices.

Après incubation on récupère les membranes par filtration sur des filtres Whatman GF/B™ traités avec de la polyéthylèneimine à 0,05 % au moyen d'un appareil Skatron Cell Harvester™, et on les lave avec environ 2,5 ml de tampon Tris-HCl (pH = 7,7 à 0°C) glacé.

On détermine la liaison non spécifique avec de l'halopéridol 1µM, on analyse les données selon les méthodes usuelles, et on calcule pour chaque composé la concentration $CI_{50}$, qui inhibe de 50 % la liaison du [$^3$H]-(+)-3PPP.
Les $CI_{50}$ (en µM) des composés de l'invention se situent entre 0,001 et 0,2 µM.

Enfin la toxicité aiguë des composés de l'invention a été évaluée chez la souris. Les doses léthales $DL_{50}$, doses qui provoquent 50 % de mortalité des animaux, se situent entre 30 et 300 mg/kg par voie intrapéritonéale, et entre 100 et 1000 mg/kg par voie orale.

Les résultats des essais montrent que, in vitro, les composés déplacent le [$^3$H]-(+)-3-PPP de ses sites de liaisons cérébrales et par conséquent présentent une affinité pour les sites sigma.
In vivo, ils possèdent des propriétés antiischémiques et anticonvulsivantes.

Ils peuvent être utilisés pour le traitement ou la protection vis-à-vis des ischémies cérébrales d'origines diverses telle que hypoglycémiques, hypoxiques et, en général, dans le traitement des encéphalopathies métaboliques, dans la protection vis à vis de la dégénérescence neuronale ainsi que des amino-acidopathies impliquant un désordre de la fonction neuronale, et dans le traitement des maladies neurologiques. Ils peuvent aussi être utilisés pour les traitements des désordres neurologiques tels que les états convulsifs en général, l'épilepsie et la spasticité en particulier, les états de stress et d'anxiété, les états psychotiques en général et

la schizophrénie.
A cet effet ils peuvent être présentés sous toutes formes appropriées à leur administration par voie orale ou parentérale, associés à tous excipients convenables, et dosés pour permettre une posologie journalière de 1 à 1000 mg.

# ANNEXE

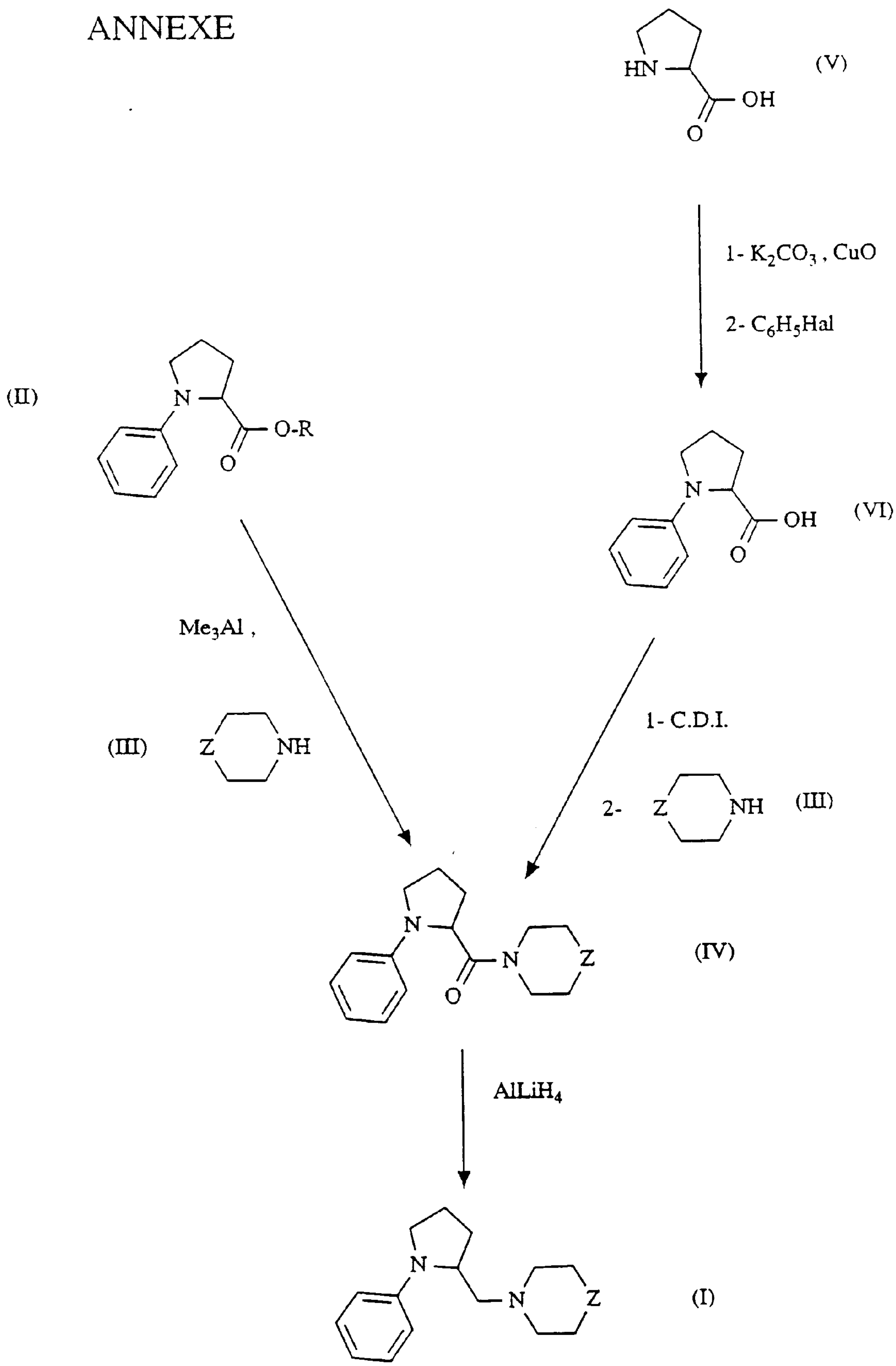


(V)

(VI)

(II)

(III)

(III)

(IV)

(I)

## Revendications

**Revendications pour les Etats contractants suivants : AT, BE, CH, DE, DK, FR, GB, IT, LI, LU, NL, SE**

**1.** Dérivés de [(1-arylpyrrolidin-2-yl)méthyl]pipérazine, sous la forme de racémates ou d'énantiomères, répondant à la formule

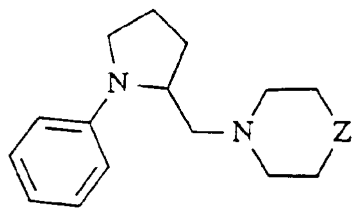

(I)

dans laquelle
Z représente un groupe de formule N-$R_1$ dans laquelle $R_1$ est soit un atome d'hydrogène, soit un groupe $(C_{1-3})$alkyle, soit un groupe de formule Ar-$C_nH_{2n}$ dans laquelle n représente 0,1 ou 2 et Ar représente un groupe phényle éventuellement substitué par un ou plusieurs atomes d'halogène et/ou radicaux $(C_{1-3})$alkyles ou $(C_{1-3})$alcoxy, soit un groupe de formule $COR_2$ dans laquelle $R_2$ représente le groupe $CH_3$, $C_6H_5$, $CH_2C_6H_5$ ou $OC_2H_5$
ainsi que leurs sels d'addition aux acides pharmaceutiquement acceptables.

**2.** Dérivés selon la revendication 1, dans lesquels Z représente un radical N-$(CH_2)_2$-Ar où Ar représente un groupe phényle éventuellement substitué par un ou plusieurs atomes de fluor ou de chlore et/ou par un ou plusieurs radicaux méthyle ou méthoxy.

**3.** Médicament caractérisé en ce qu'il consiste en un composé selon l'une quelconque des revendications 1 et 2.

**4.** Composition pharmaceutique caractérisée en ce qu'elle contient un composé selon l'une quelconque des revendications 1 et 2, associé à un excipient convenable.

**5.** Procédé de préparation des composés selon la revendication 1, procédé caractérisé en ce que

. soit on fait réagir, dans un solvant inerte tel que le toluène, à une température allant de 80 à 110°C, un ester dérivé de la proline de formule (II)

(II)

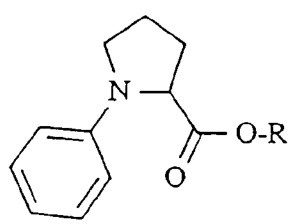

avec le complexe triméthylaluminium-amine de formule (III),

(III) 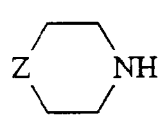 , $Me_3Al$

préparé au préalable dans un solvant inerte tel que le toluène, à une température allant de -20 à 20°C pour obtenir le composé (IV)

. soit on fait réagir la proline (V)

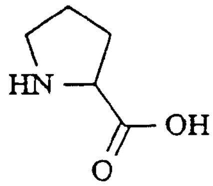

(V)

avec un halogénure $C_6H_5Hal$ en présence soit de sel de Cu++ sous forme de CuO, soit de cuivre métallique, en milieu basique, par exemple $K_2CO_3$, dans un solvant aprotique, tel que le DMF ou la N-méthylpyrrolidone, à une température allant de 100 à 150°C, pour obtenir un acide de formule (VI) ;

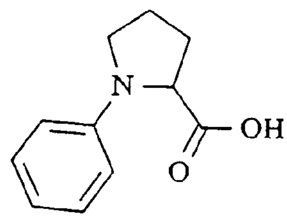

(VI)

puis on effectue la transformation de l'acide (VI) en amide (IV), en traitant l'acide (VI) au moyen de carbonyldiimidazole dans un solvant, tel que le tétrahydrofurane (THF) ou le chlorure de méthylène ($CH_2Cl_2$), à une température allant de 20 à 40°C pour former l'imidazolide intermédiaire que l'on traite in situ par une amine de formule (III)
enfin on réduit l'amide obtenu de formule (IV)

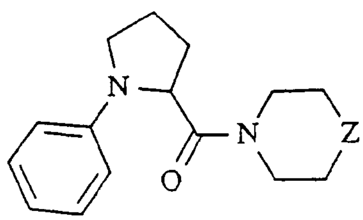

(IV)

au moyen d'hydrure de lithium et d'aluminium dans un solvant éthéré tel que l'éther ou le tétrahydrofurane (THF) à une température allant de 20 à 50°C en composé de formule (I).

**Revendications pour les Etats contractants suivants : ES, GR**

1. Procédé de préparation de dérivés de [(1-arylpyrrolidin-2-yl)méthyl]pipérazine, sous la forme de racémates ou d'énantiomères, répondant à la formule

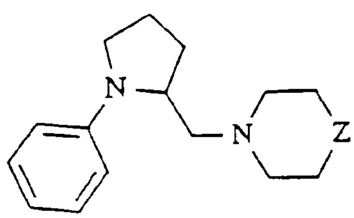

(I)

dans laquelle
Z représente un groupe de formule N-$R_1$ dans laquelle $R_1$ est soit un atome d'hydrogène, soit un groupe ($C_1$-$_3$)alkyle, soit un groupe de formule Ar-$C_nH_{2n}$ dans laquelle n représente 0,1 ou 2 et Ar représente un groupe phényle éventuellement substitué par un ou plusieurs atomes d'halogène et/ou radicaux ($C_{1-3}$)alkyles ou ($C_{1-3}$)alcoxy, soit un groupe de formule $COR_2$ dans laquelle $R_2$ représente le groupe $CH_3$, $C_6H_5$, $CH_2C_6H_5$ ou $OC_2H_5$,

ainsi que de leurs sels d'addition aux acides pharmaceutiquement acceptables, procédé caractérisé en ce que

. soit on fait réagir, dans un solvant inerte tel que le toluène, à une température allant de 80 à 110°C, un ester dérivé de la proline de formule (II)

(II) 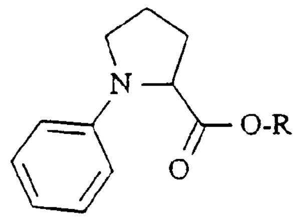

avec le complexe triméthylaluminium-amine de formule (III),

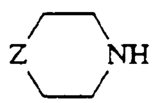 (III) , $Me_3Al$

préparé au préalable dans un solvant inerte tel que le toluène, à une température allant de -20 à 20°C pour obtenir le composé (IV)

. soit on fait réagir la proline (V)

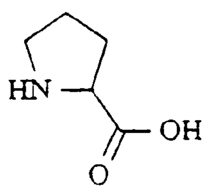 (V)

avec un halogénure $C_6H_5Hal$ en présence soit de sel de Cu++ sous forme de CuO, soit de cuivre métallique, en milieu basique, par exemple $K_2CO_3$, dans un solvant aprotique, tel que le DMF ou la N-méthylpyrrolidone, à une température allant de 100 à 150°C, pour obtenir un acide de formule (VI) ;

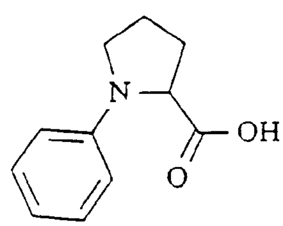 (VI)

puis on effectue la transformation de l'acide (VI) en amide (IV), en traitant l'acide (VI) au moyen de carbonyldiimidazole dans un solvant, tel que le tétrahydrofurane (THF) ou le chlorure de méthylène ($CH_2Cl_2$), à une température allant de 20 à 40°C pour former l'imidazolide intermédiaire que l'on traite in situ par une amine de formule (III) dans laquelle Z a la signification donnée ci-dessus

enfin on réduit l'amide obtenu de formule (IV)

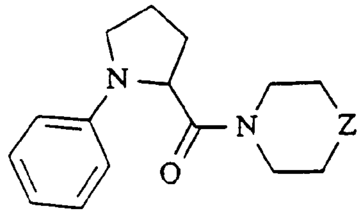

(IV)

au moyen d'hydrure de lithium et d'aluminium dans un solvant éthéré tel que l'éther ou le tétrahydrofurane (THF) à une température allant de 20 à 50°C en composé de formule (I).

2. Procédé de préparation selon la revendication 1, procédé caractérisé en ce que l'on prépare les composés (I) dans lesquels
Z représente un radical $N-(CH_2)_2-Ar$ où Ar représente un groupe phényle éventuellement substitué par un ou plusieurs atomes de fluor ou de chlore et/ou par un ou plusieurs radicaux méthyle ou méthoxy.

## Patentansprüche

### Patentansprüche für folgende Vertragsstaaten : AT, BE, CH, DE, DK, FR, GB, IT, LI, LU, NL, SE

1. [(1-Arylpyrrolidin-2-yl)-methyl]-piperazin-Derivate in Form der Racemate oder der Enantiomeren, der allgemeinen Formel (I)

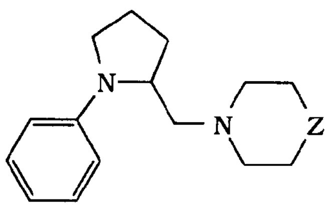

(I)

in der
Z eine Gruppe der Formel $N-R_1$ bedeutet, worin $R_1$ entweder ein Wasserstoffatom oder eine $(C_{1-3})$-Alkylgruppe oder eine Gruppe der Formel $Ar-C_nH_{2n}$, in der n 0, 1 oder 2 und Ar eine Phenylgruppe, die gegebenenfalls durch eines oder mehrere Halogenatome und/oder $(C_{1-3})$-Alkylgruppen oder $(C_{1-3})$-Alkoxygruppen substituiert ist, darstellen. oder eine Gruppe der Formel $COR_2$ bedeutet, in der $R_2$ die Gruppe $CH_3$, $C_6H_5$, $CH_2C_6H_5$ oder $OC_2H_5$ darstellt, sowie deren Additionssalze mit pharmazeutisch annehmbaren Säuren.

2. Derivate nach Anspruch 1, worin Z eine Gruppe $N-(CH_2)_2-Ar$ bedeutet, in der Ar eine Phenylgruppe darstellt, die gegebenenfalls durch eines oder mehrere Fluor- oder Chloratome und/oder durch eine oder mehrere Methyl- oder Methoxygruppen substituiert ist.

3. Arzneimittel, **dadurch gekennzeichnet,** daß es aus einer Verbindung nach einem der Ansprüche 1 und 2 besteht.

4. Pharmazeutische Zubereitung, **dadurch gekennzeichnet,** daß sie eine Verbindung nach einem der Ansprüche 1 und 2 in Kombination mit einem geeigneten Trägermaterial enthält.

5. Verfahren zur Herstellung der Verbindunge nach Anspruch 1, **dadurch gekennzeichnet,** daß man
   - entweder einen von Prolin abgeleiteten Ester der Formel (II)

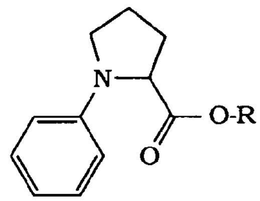

(II)

in einem inerten Lösungsmittel, wie Toluol, bei einer Temperatur von 80 bis 110°C mit dem Trimethylaluminium-Amin-Komplex der Formel (III)

(III) 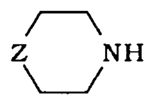 , $Me_3Al$

den man zuvor in einem inerten Lösungsmittel, wie Toluol, bei einer Temperatur von -20 bis 20°C hergestellt hat, umsetzt zur Bildung der Verbindung (IV),

- oder Prolin (V)

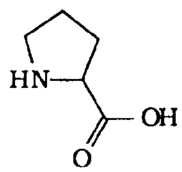

(V)

mit einem Halogenid $C_6H_5Hal$ in Gegenwart von entweder einem Kupfer$^{++}$-Salz in Form von CuO oder von metallischem Kupfer, in basischem Medium, beispielsweise $K_2CO_3$, in einem aprotischen Lösungsmittel, wie DMF oder N-Methylpyrrolidon, bei einer Temperatur von 100 bis 150°C umsetzt zur Bildung einer Säure der Formel (VI):

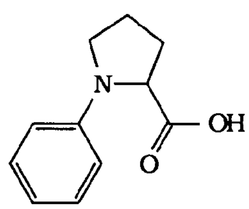

(VI)

und dann die Umwandlung der Säure (VI) in das Amid (IV) bewirkt, indem man die Säure (VI) mit Carbonyldiimidazol in einem Lösungsmittel, wie Tetrahydrofuran (THF) oder Methylenchlorid ($CH_2Cl_2$), bei einer Temperatur von 20 bis 40°C zur Bildung des Imidazolid-Zwischenprodukts behandelt, welches man in situ mit einem Amin der Formel (III) behandelt, wonach man das erhaltene Amid der Formel (IV)

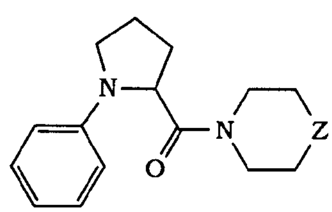

(IV)

mit Lithiumaluminiumhydrid in einem Etherlösungsmittel, wie Ether oder Tetrahydrofuran (THF) bei einer Temperatur von 20 bis 50°C zu der Verbindung der Formel (I) reduziert.

**Patentansprüche für folgende Vertragsstaaten : ES, GR**

1. Verfahren zur Herstellung von [(1-Arylpyrrolidin-2-yl)-methyl]-piperazin-Derivaten in Form der Racemate oder der Enantiomeren der Formel (I)

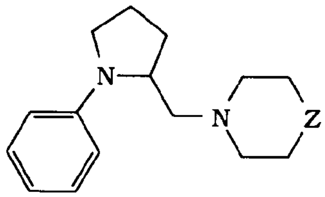

(I)

in der
Zeine Gruppe der Formel N-$R_1$ bedeutet, worin $R_1$ entweder ein Wasserstoffatom oder eine ($C_{1-3}$)-Alkylgruppe oder eine Gruppe der Formel Ar-$C_nH_{2n}$, in der n 0, 1 oder 2 und Ar eine Phenylgruppe, die gegebenenfalls durch eines oder mehrere Halogenatome und/oder ($C_{1-3}$)-Alkylgruppen oder ($C_{1-3}$)-Alkoxygruppen substituiert ist, darstellen, oder eine Gruppe der Formel $COR_2$ bedeutet, in der $R_2$ die Gruppe $CH_3$, $C_6H_5$, $CH_2C_6H_5$ oder $OC_2H_5$ darstellt, sowie von deren Additionssalzen mit pharmazeutisch annehmbaren Säuren, **dadurch gekennzeichnet,** daß man

- entweder einen von Prolin abgeleiteten Ester der Formel (II)

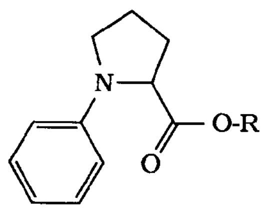

(II)

in einem inerten Lösungsmittel, wie Toluol, bei einer Temperatur von 80 bis 110°C mit dem Trimethylaluminium-Amin-Komplex der Formel (III)

(III) 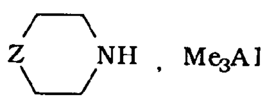

den man zuvor in einem inerten Lösungsmittel, wie Toluol, bei einer Temperatur von -20 bis 20°C hergestellt hat, umsetzt zur Bildung der Verbindung (IV),

- oder Prolin (V)

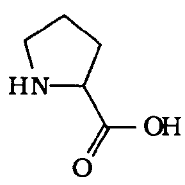

(V)

mit einem Halogenid $C_6H_5$Hal in Gegenwart von entweder einem Kupfer$^{++}$-Salz in Form von CuO oder von metallischem Kupfer, in basischem Medium, beispielsweise $K_2CO_3$, in einem aprotischen Lösungsmittel, wie DMF oder N-Methylpyrrolidon, bei einer Temperatur von 100 bis 150°C umsetzt zur Bildung einer Säure der Formel (VI):

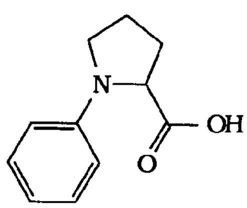

(VI)

und dann die Umwandlung der Säure (VI) in das Amid (IV) bewirkt, indem man die Säure (VI) mit Carbonyldiimidazol in einem Lösungsmittel, wie Tetrahydrofuran (THF) oder Methylenchlorid ($CH_2Cl_2$), bei einer Temperatur von 20 bis 40°C zur Bildung des Imidazolid-Zwischenprodukts behandelt, welches man in situ mit einem Amin der Formel (III) behandelt, wonach man das erhaltene Amid der Formel (IV)

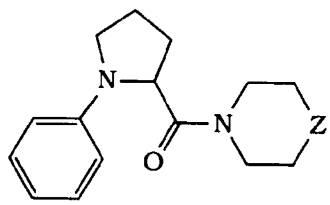

(IV)

mit Lithiumaluminiumhydrid in einem Etherlösungsmittel, wie Ether oder Tetrahydrofuran (THF) bei einer Temperatur von 20 bis 50°C zu der Verbindung der Formel (I) reduziert.

2. Verfahren nach Anspruch 1, **dadurch gekennzeichnet,** daß man Verbindungen (I) herstellt, worin Z eine Gruppe N-$(CH_2)_2$-Ar bedeutet, in der Ar eine Phenylgruppe darstellt, die gegebenenfalls durch eines oder mehrere Fluor- oder Chloratome und/oder durch eine oder mehrere Methyl- oder Methoxygruppen substituiert ist.

## Claims

**Claims for the following Contracting States : AT, BE, CH, DE, DK, FR, GB, IT, LI, LU, NL, SE**

1. [(1-Arylpyrrolidin-2-yl)methyl]piperazine derivatives in the form of racemates or enantiomers, corresponding to the formula

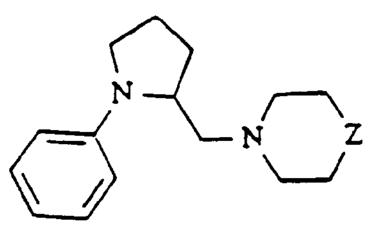

(I)

in which
Z denotes a group of formula N-$R_1$ in which $R_1$ is either a hydrogen atom or a $C_{1-3}$ alkyl group or a group of formula Ar-$C_nH_{2n}$ in which n denotes 0, 1 or 2 and Ar denotes a phenyl group optionally substituted by one or more halogen atoms and/or $C_{1-3}$ alkyl or $C_{1-3}$ alkoxy radicals, or a group of formula $COR_2$ in which $R_2$ denotes the $CH_3$, $C_6H_5$, $CH_2C_6H_5$ or $OC_2H_5$ group
and their salts of addition to pharmaceutically acceptable acids.

2. Derivatives according to Claim 1, in which Z denotes an N-$(CH_2)_2$-Ar radical where Ar denotes a phenyl group optionally substituted by one or more fluorine or chlorine atoms and/or by one or more methyl or methoxy radicals.

3. Medication characterized in that it consists of a compound according to either of Claims 1 and 2.

4. Pharmaceutical composition characterized in that it contains a compound according to either of Claims 1 and 2, in combination with a suitable excipient.

5. Process for the preparation of the compounds according to Claim 1, a process characterized in that either an ester derived from proline, of formula (II)

(II) 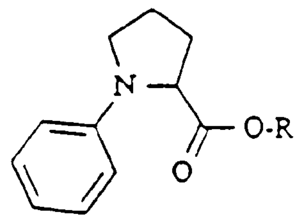

is reacted, in an inert solvent such as toluene, at a temperature ranging from 80 to 110°C, with the trimethylaluminium-amine complex of formula (III)

(III) 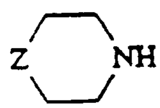 , $Me_3Al$

prepared beforehand in an inert solvent such as toluene, at a temperature ranging from -20 to 20°C, to obtain the compound (IV)

or proline (V)

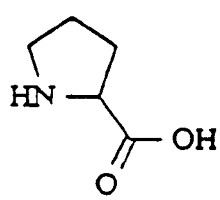 (V)

is reacted with a halide $C_6H_5Hal$ in the presence either of a $Cu^{++}$ salt in the form of CuO, or of metallic copper, in a basic medium, for example $K_2CO_3$, in an aprotic solvent such as DMF or N-methylpyrrolidone, at a temperature ranging from 100 to 150°C, to obtain an acid of formula (VI);

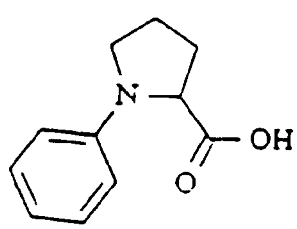 (VI)

the acid (VI) is then converted into amide (IV), by treating the acid (VI) with carbonyldiimidazole in a solvent such as tetrahydrofuran (THF) or methylene chloride ($CH_2Cl_2$) at a temperature ranging from 20 to 40°C, to form the intermediate imidazolide which is treated in situ with an amine of formula (III)

finally, the amide, of formula (IV), which is obtained

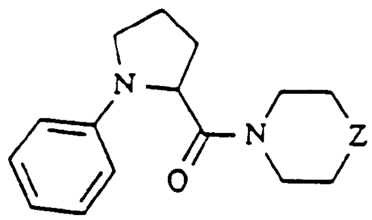

(IV)

is reduced with lithium aluminium hydride in an ether solvent such as ether or tetrahydrofuran (THF) at a temperature ranging from 20 to 50°C to a compound of formula (I).

**Claims for the following Contracting States : ES, GR**

**1.** Process for the preparation of [(1-aryl-pyrrolidin-2-yl)methyl]piperazine derivatives in the form of racemates or enantiomers, corresponding to the formula

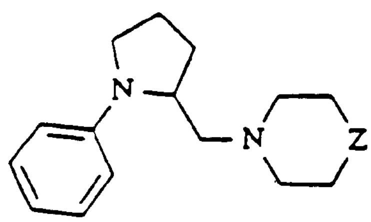

(I)

in which
Z denotes a group of formula $N-R_1$ in which $R_1$ is either a hydrogen atom or a $C_{1-3}$ alkyl group or a group of formula $Ar-C_nH_{2n}$ in which n denotes 0, 1 or 2 and Ar denotes a phenyl group optionally substituted by one or more halogen atoms and/or $C_{1-3}$ alkyl or $C_{1-3}$ alkoxy radicals, or a group of formula $COR_2$ in which $R_2$ denotes the $CH_3$, $C_6H_5$, $CH_2C_6H_5$ or $OC_2H_5$ group,
and of their salts of addition to pharmaceutically acceptable acids, a process characterized in that

either an ester derived from proline, of formula (II)

(II)

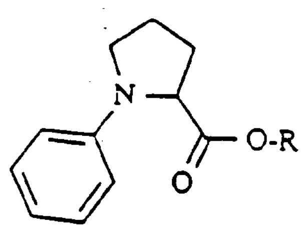

is reacted, in an inert solvent such as toluene, at a temperature ranging from 80 to 110°C, with the trimethylaluminium-amine complex of formula (III)

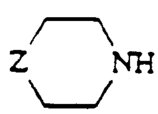

(III) , $Me_3Al$

prepared beforehand in an inert solvent such as toluene, at a temperature ranging from -20 to 20°C, to obtain the compound (IV)

or proline (V)

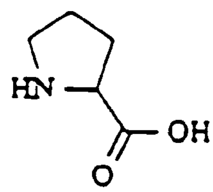

(V)

is reacted with a halide $C_6H_5Hal$ in the presence either of a $Cu^{++}$ salt in the form of CuO, or of metallic copper, in a basic medium, for example $K_2CO_3$, in an aprotic solvent such as DMF or N-methylpyrrolidone, at a temperature ranging from 100 to 150°C, to obtain an acid of formula (VI);

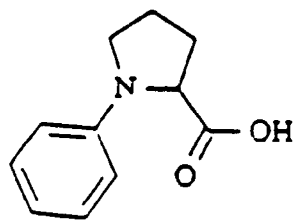

(VI)

the acid (VI) is then converted into amide (IV), by treating the acid (VI) with carbonyldiimidazole in a solvent such as tetrahydrofuran (THF) or methylene chloride ($CH_2Cl_2$) at a temperature ranging from 20 to 40°C, to form the intermediate imidazolide which is treated in situ with an amine of formula (III) in which Z has the meaning given above;
finally, the amide, of formula (IV) which is obtained

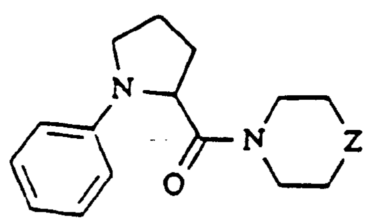

(IV)

is reduced with lithium aluminium hydride in an ether solvent such as ether or tetrahydrofuran (THF) at a temperature ranging from 20 to 50°C to a compound of formula (I).

2. Preparation process according to Claim 1, a process characterized in that the compounds (I) are prepared in which
Z denotes an N-$(CH_2)_2$-Ar radical where Ar denotes a phenyl group optionally substituted by one or more fluorine or chlorine atoms and/or by one or more methyl or methoxy radicals.